(19) Europäisches Patentamt
European Patent Office
Office européen des brevets

(11) EP 3 588 090 A1

(12) EUROPEAN PATENT APPLICATION
published in accordance with Art. 153(4) EPC

(43) Date of publication:
**01.01.2020 Bulletin 2020/01**

(21) Application number: **18757368.8**

(22) Date of filing: **20.02.2018**

(51) Int Cl.:
***G01N 33/66*** *(2006.01)* ***G01N 27/62*** *(2006.01)*
***G01N 30/06*** *(2006.01)* ***G01N 30/88*** *(2006.01)*
***G01N 33/48*** *(2006.01)*

(86) International application number:
**PCT/JP2018/006043**

(87) International publication number:
**WO 2018/155443 (30.08.2018 Gazette 2018/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **21.02.2017 JP 2017030518**
**10.10.2017 JP 2017196506**

(71) Applicant: **JCR Pharmaceuticals Co., Ltd.**
**Ashiya-shi**
**Hyogo 659-0021 (JP)**

(72) Inventors:
- **TANAKA, Noboru**
**Kobe-shi**
**Hyogo 651-2241 (JP)**
- **KIDA, Sachiho**
**Kobe-shi**
**Hyogo 651-2241 (JP)**

(74) Representative: **Balder IP Law, S.L.**
**Paseo de la Castellana 93**
**5a planta**
**28046 Madrid (ES)**

(54) **METHOD FOR ANALYZING GLYCOSAMINOGLYCAN**

(57) [Problem] A method for measuring the amount of trace amo unts of heparan sulfate and/or dermatan sulfate containe d in a sample is provided.

[Solution] The present invention provides a method for decomposing heparan sulfate and dermatan sulfate contained in a sample into, respectively, a disaccharide in which uronic acid and amino sugar are α1,4-bonded and a disaccharide in which uronic acid and amino sugar are α1,3-bonded. More particularly, the present invention provides a method for decomposing heparan sulfate and dermatan sulfate by heating the heparan sulfate and the dermatan sulfate in, for example, 2,2-dimethoxypropane-containing methanol hydrochloride at 65-85°C for 80-180 minutes and 60-80°C for 20-100 minutes, respectively.

EP 3 588 090 A1

## Description

[TECHNICAL FIELD]

The present invention relates to a method for decomposing heparan sulfate or/and dermatan sulfate to disaccharides, and further to a method for determining the amount of heparan sulfate or/and dermatan sulfate by analyzing the disaccharides by liquid chromatography-mass spectrometry.

[BACKGROUND ART]

Glycosaminoglycans (GAGs) are a group of acidic polysaccharides containing amino acids therein. GAG has a long chain structure in which a disaccharide consisting of an amino sugar (glucosamine, galactosamine) and uronic acid (glucuronic acid, etc.) or galactose are repeatedly linked. Some sugars make up GAG are sulfated. GAGs include hyaluronic acid, chondroitin 4-sulfate, chondroitin 6-sulfate, heparin, heparan sulfate, dermatan sulfate, and keratan sulfate.

In each type of GAG, there are enzymes that specifically degrade them. Some genetic diseases are caused by genetic deletion of these enzymes.

For example, iduronic acid-2-sulfatase (IDS) is an enzyme having an activity of hydrolyzing a sulfate ester bond existing in a molecule such as heparan sulfate or dermatan sulfate. The loss of some or all of the activity of this enzyme genetically leads to Hunter syndrome. In patients with Hunter syndrome, dermatan sulfate and heparan sulfate accumulate in various tissues, causing symptoms such as skeletal deformity and severe mental retardation.

$\alpha$-L-iduronidase (IDUA) is an enzyme having an activity of hydrolyzing iduronic acid bonds present in molecules such as dermatan sulfate and heparan sulfate. The loss of some or all of the activity of this enzyme genetically results in Hurler syndrome. Scheie syndrome is a mild form of Hurler syndrome, and Hurler-Scheie syndrome is an intermediate form of these, all of which are caused by a genetic defect in $\alpha$-L-iduronidase. In patients with Hurler syndrome, the accumulation of dermatan sulfate and heparan sulfate in tissues causes symptoms such as severe physical deformity and mental retardation.

N-acetylgalactosamine-4-sulfatase (ASB) is an enzyme that hydrolyzes chondroitin 4-sulfate, dermatan sulfate, and the like to liberate sulfate ions. The loss of some or all of the activity of this enzyme genetically results in Maroteaux-Lamy syndrome. In patients with Maroteaux-Lamy syndrome, the accumulation of dermatan sulfate and the like in the tissues causes various symptoms such as growth retardation and marked deformation of the extremities and spine.

In addition, Sanfilippo syndrome, Morquio syndrome, Sly syndrome, and the like are known as diseases caused by genetic defects of enzymes that degrade GAG.

Diseases caused by deficiencies in enzymes that degrade GAG are collectively referred to as mucopolysaccharidosis. GAGs accumulate abnormally in the tissues of patients with mucopolysaccharides. Thus, the effect of an agent for indication of mucopolysaccharidosis can be quantitatively determined, for example, by measuring changes in the amount of GAG present in the tissue. Also, by measuring the amount of GAG present in the tissue, patients with mucopolysaccharidosis can be identified.

As a method for analyzing GAG, a method is known in which GAG contained in a sample is decomposed by a reductive amination reaction to obtain a fluorescently labeled disaccharide, which is analyzed by liquid chromatography (Patent Document 1). In addition, a method of decomposing GAG contained in a sample to disaccharides by a methanolysis treatment and analyzing the disaccharides by using a mass spectrometer directly connected to liquid chromatography is known (Non-Patent Documents 1 to 3).

[PRIOR-ART DOCUMENTS]

[PATENT DOCUMENT]

[Patent Document 1] JP 2012-108056

[NON-PATENT DOCUMENTS]

[Non-Patent Document 1] Auray-Blais C.et al., Mol Genet Metab. 102. 49-56 (2011)
[Non-Patent Document 2] Auray-Blais C.et al., Clin Chim Acta. 413. 771-8 (2012)
[Non-Patent Document 3] Zang H.et al., Clin Chem. 57. 1005-12 (2011)

[SUMMARY OF INVENTION]

[TECHNICAL PROBLEM]

It is an object of the present invention to provide a method for decomposing heparan sulfate or/and dermatan sulfate to disaccharides constituting the same, and a method for analyzing and measuring disaccharides obtained by decomposing heparan sulfate or/and dermatan sulfate by liquid chromatography-mass spectrometry.

[TECHNICAL SOLUTION]

In the studies directed to the above-mentioned objects, as a result of intensive studies, the present inventors have found that heparan sulfate and dermatan sulfate constituting GAG can be efficiently decomposed to disaccharides by decomposing them in the presence of hydrogen chloride methanol solution, and that heparan sulfate and dermatan sulfate obtained by decomposition can be measured sensitively by analyzing disaccharides obtained by liquid chromatography-mass spectrometry, thereby completing the present invention. That is, the present invention includes the following.

1. A method of decomposing heparan sulfate contained in a sample to a disaccharide in which a uronic acid and an amino sugar are joined with an α-1,4 bond, wherein the heparan sulfate is decomposed by heating in hydrogen chloride methanol solution containing 2,2-dimethoxypropane, for 80 to 180 minutes, and at a temperature of 65 to 85°C.
2. The method of 1 above, wherein the heating is performed for 110 to 130 minutes and at a temperature of 78 to 82°C.
3. The method of 1 or 2 above, wherein the sample is selected from or derived from any one of a bodily fluid, a cell, a tissue, an organ, a cell culture, a tissue culture, a food, and a feedstuff.
4. The method of 1 or 2 above, wherein the sample is selected from or derived from any one of a bodily fluid, a cell, a tissue, an organ, a blood, a serum, a plasma, a urine, a bone marrow fluid, and a cerebrospinal fluid, obtained from a mammal.
5. The method of 4 above, wherein the mammal is selected from the group consisting of a human, a monkey, a mouse, a rat, a guinea pig, a hamster, a rabbit, a horse, a bovine, a pig, a dog, and a cat.
6. The method of 4 above, wherein the mammal is a human, and the human is a patient with a disease in which the heparan sulfate accumulates in a body.
7. The method of claim 6 above, wherein the disease is selected from the group consisting of Hunter syndrome, Hurler syndrome, Scheie syndrome, Hurler-Scheie syndrome, Sanfilippo syndrome, and Sly syndrome.
8. The method of 6 or 7 above, wherein the patient has been treated to reduce the heparan sulfate accumulated in the body.
9. A method for decomposing dermatan sulfate and heparan sulfate contained in a sample into a disaccharide, respectively, wherein
the dermatan sulfate is decomposed into a disaccharide in which a uronic acid and an amino sugar are joined with an α-1,3 bond, and the heparan sulfate is decomposed into the disaccharide in which the uronic acid and the amino sugar are joined with the α-1,4 bond, and wherein,
the dermatane sulfate is decomposed by heating in hydrogen chloride methanol solution containing 2,2-dimethoxypropane, at a temperature of 60 to 80°C, and for 20 to 100 minutes, and
the heparan sulfate is decomposed by the method of 1 or 2 above.
10. The method of 9 above, wherein the heating for the decomposition of the dermatan sulfate is carried out at a temperature of 63 to 67°C and for 30 to 80 minutes.
11.The method of 9 or 10 above, wherein the sample is selected from or derived from any one of a bodily fluid, a cell, a tissue, an organ, a cell culture, a tissue culture, a food, and a feedstuff.
12. The method of 9 or 10 above, wherein the sample is selected from or derived from the group consisting of a bodily fluids, a cell, a tissue, an organ, a blood, a serum, a plasma, a urine, a bone marrow fluid, and a cerebrospinal fluid, obtained from a mammal.
13. The method of 12 above, wherein the mammal is selected from the group consisting of a human, a monkey, a mouse, a rat, a guinea pig, a hamster, a rabbit, a horse, a bovine, a pig, a dog, and a cat.
14. The method of 12 above, wherein the mammal is a human, and the human is a patient with a disease in which the heparan sulfate or/and the dermatan sulfate accumulate in a body.
15. The method of 14 above, wherein the disease is selected from the group consisting of Hunter syndrome, Hurler syndrome, Scheie syndrome, Hurler-Scheie syndrome, Maroteaux-Lamy syndrome, Sanfilippo syndrome, and Sly syndrome.

16. The method of 14 or 15 above, wherein the patient has been treated to reduce the heparan sulfate or/and the dermatan sulfate accumulated in the body.
17. A method for measuring the amount of the heparan sulfate in the sample, the method comprising: the step of subjecting the disaccharide obtained by any one of the methods of 1 to 8 above to liquid chromatography to obtain an eluate, and the step of subjecting the eluate to mass spectrometry.
18. A method for determining the amounts of the heparan sulfate and the dermatan sulfate in the sample, the method comprising:

the step of subjecting the disaccharide obtained, by any one of the methods of 9 to 16 above, by decomposing the dermatan sulfate and the heparan sulfate, respectively, to obtain an eluate, and
the step of subjecting the eluate to mass spectrometry.

19. A method of detecting an individual with a disease in which heparan sulfate or/and dermatan sulfate accumulate in a body from among mammals from which the sample is provided, based on a measured value, obtained by the method of 17 or 18 above, of the heparan sulfate or/and the dermatan sulfate contained in the sample.
20. The method of 19 above, wherein the disease is selected from the group consisting of Hunter syndrome, Hurler syndrome, Scheie syndrome, Hurler-Scheie syndrome, Maroteaux-Lamy syndrome, Sanfilippo syndrome, and Sly syndrome.
21. A method of confirming the effect of a treatment for decreasing heparan sulfate and dermatan sulfate accumulated in a body, based on a measured value obtained, by the method of 17 or 18 above, by measuring the amount of the heparan sulfate or/and the dermatan sulfate contained in a sample, the sample is obtained from a patient suffering from a disease in which heparan sulfate and dermatan sulfate accumulate in the body before and after the treatment, respectively.

[EFFECT OF INVENTION]

According to the present invention, for example, heparan sulfate and/or dermatan sulfate contained in a sample taken from a mammal (serum, cerebrospinal fluid, etc.) can be measured sensitively.

[BRIEF DESCRIPTION OF DRAWINGS]

Figure 1 indicates an ion chromatogram, obtained by LC/MS/MS spectrometry, of a solution for preparing a calibration curve containing 25 ng/mL each of dermatan sulfate and heparan sulfate, that solution was subjected to methanolysis under the condition A shown in Table 1. The vertical axis represents counts per second (cps), and the horizontal axis represents elution time (minutes) .
Figure 2 indicates an ion chromatogram, obtained by LC/MS/MS spectrometry, of a solution for preparing a calibration curve containing 25 ng/mL each of dermatan sulfate and heparan sulfate, that solution was subjected to methanolysis under the condition B shown in Table 1. The vertical axis represents counts per second (cps), and the horizontal axis represents elution time (minutes).
Figure 3 shows the results of measurement of the concentration of dermatan sulfate contained in the serum of mice. The vertical axis represents the concentration (μg/mL) of dermatan sulfate in mouse serum. White bars indicate the concentration of dermatan sulfate in the serum of wild-type mice and black bars indicate the concentration of dermatan sulfate in the serum of IDS hemizygote mice. Error bars indicate standard deviation (n=3). (A) shows the results of measurement of 75 minutes, 65°C, (B) shows the results of measurement of 2 hours, 80°C, and (C) shows the results of methanolysis processing at 18 hours and 65°C, respectively.
Figure 4 shows the results of measurement of the concentration of heparan sulfate contained in the serum of mice. The vertical axis represents the concentration (μg/mL) of heparan sulfate in mouse serum. White bars indicate the results of the concentration of heparan sulfate in the serum of wild-type mice and black bars indicate the results of the concentration of heparan sulfate in the serum of IDS hemizygous mice. Error bars indicate standard deviation (n=3). (A) shows the results of measurement of 75 minutes, 65°C, (B) shows the results of measurement of 2 hours, 80°C, and (C) shows the results of methanolysis processing at 18 hours and 65°C, respectively.
Figure 5 shows the results of measuring the concentrations of heparan sulfate and dermatan sulfate contained in the sera of mice treated with rhIDS. The vertical axis represents the concentration of heparan sulfate and dermatan sulfate in mouse serum (μg/mL). White bar, black bars, and shaded bars represent the results of wild-type mice, IDS hemizygotes without rhIDS, and IDS hemizygotes with rhIDS, respectively. Error bars indicate standard deviations, double # indicates $p<0.01$ in the t-test (comparison between wild-type mice and IDS hemizygote mice not-administered with rhIDS), and double asterisks indicate p<0.01 in the t-test (comparison between IDS hemizygote mice not-administered with rhIDS and IDS hemizygote mice administered with rhIDS). (A) shows the measurement result of the concentration of heparan sulfate, and (B) shows the measurement result of the concentration of dermatan sulfate.

[DESCRIPTION OF EMBODIMENTS]

In the present invention, the term "dermatan sulfate" means a compound containing a repeating structure of a disaccharide in which uronic acid and an amino sugar are linked with an $\alpha$-1,4 bond. Uronic acid includes, but is not limited to, L-iduronic acid, L-iduronic acid-2-sulfate, and amino sugars include N-acetyl-D-galactosamine-4-sulfate. The disaccharide serving as a unit of the repeating structure of dermatan sulfate is represented by, for example, the following formula (I).

[Chem. 1]

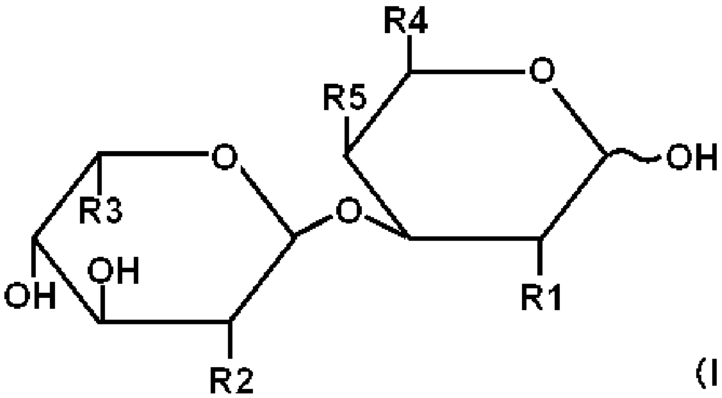

(I)

[In Formula (I), $R_1$ is $NH_2$, $NHCOCH_3$, $NHSO_3H$ or a salt thereof; $R_2$ is OH, $OSO_3H$ or a salt thereof; $R_3$ is COOH or a salt thereof; $R_4$ is $CH_2OH$,$CH_2OSO_3H$ or a salt thereof; $R_5$ is OH, $OSO_3H$, or a salt thereof, provided that any one of $R_1$, $R_2$, $R_4$ and $R_5$ contains a sulfate group]

The following formula (II) shows a more specific example of a disaccharide as a unit of the repeating structure of dermatan sulfate, in which uronic acid is L-iduronic acid and amino sugar is N-acetyl-D-galactosamine-4-sulfate.

[Chem. 2]

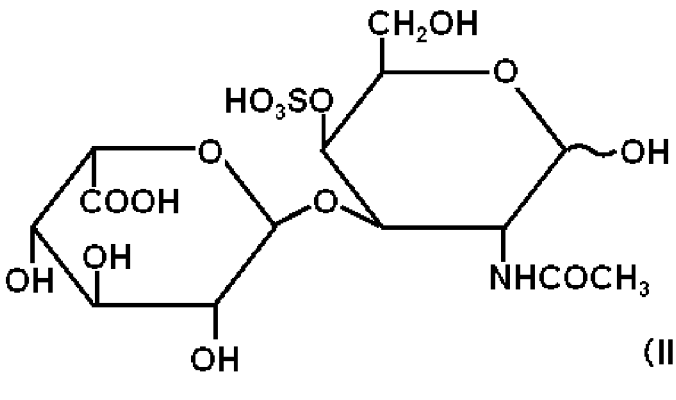

(II)

In the present invention, the term "heparan sulfate" means a compound containing a repeating structure of a disaccharide in which uronic acid and an amino sugar are joined with an $\alpha$-1,4 bond. Uronic acid includes, but is not limited to, L-iduronic acid, L-iduronic acid-2-sulfate, and amino sugars include D-glucosamine, N-sulfo-D-glucosamine-6-sulfate. The disaccharide serving as a unit of the repeating structure of heparan sulfate is represented by, for example, the following formula (III).

[Chem. 3]

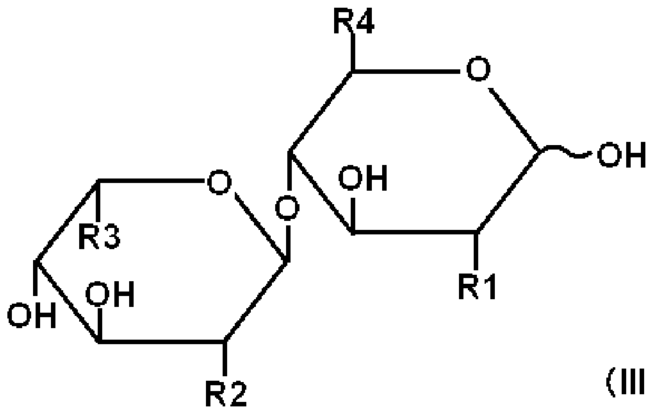

(III)

[In Formula (III), $R_1$ is $NH_2$, $NHCOCH_3$, $NHSO_3H$ or a salt thereof; $R_2$ is OH, $OSO_3H$ or a salt thereof; $R_3$ is COOH or a salt thereof; $R_4$ is $CH_2OH$,$CH_2OSO_3H$ or a salt thereof, provided that any one of $R_1$, $R_2$, and $R_4$ contains a sulfate group]

The following formula (IV) shows a more specific example of a disaccharide as a unit of the repeating structure of heparan sulfate, in which uronic acid is L-iduronic acid-2-sulfate and amino sugar is N-sulfo-D-glucosamine-6-sulfate.

[Chem. 4]

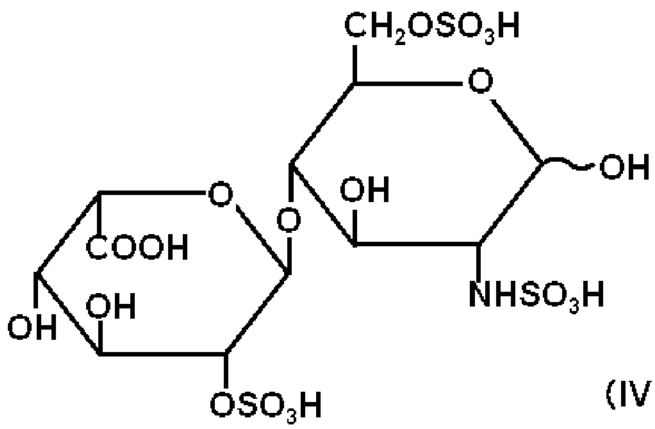

(IV)

Methanolysis is a reaction in which dermatan sulfate is decomposed in hydrogen chloride methanol solution to a disaccharide in which uronic acid and amino sugar are joined with an α-1,3 bond. The same is true for the decomposition of heparan sulfate to a disaccharide in which uronic acid and amino sugars are joined with an α-1,4 bond.

The reaction formula for the methanolysis of dermatan sulfate, in which uronic acid is L-iduronic acid and amino sugar is N-acetyl-D-galactosamine-4-sulfate, is illustrated in Formula (V) below.

[Chem. 5]

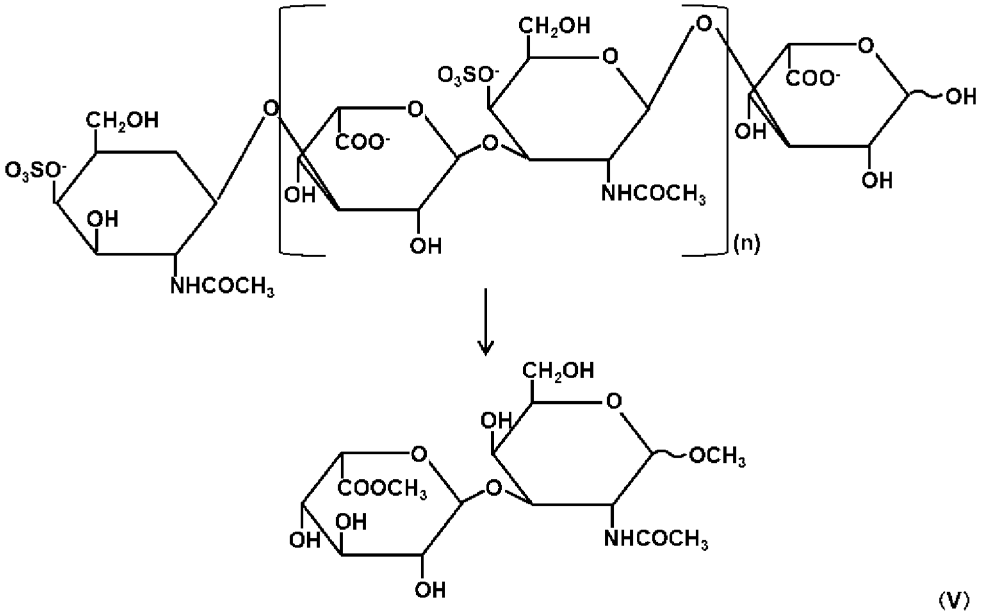

(V)

[In Formula (V), n represents an integer of 1 or more.]

The reaction scheme for the methanolysis of heparan sulfate, wherein uronic acid is L-iduronic acid-2-sulfate and amino sugar is N-sulfo-D-glucosamine-6-sulfate, is illustrated in Formula (VI) below.

[Chem. 6]

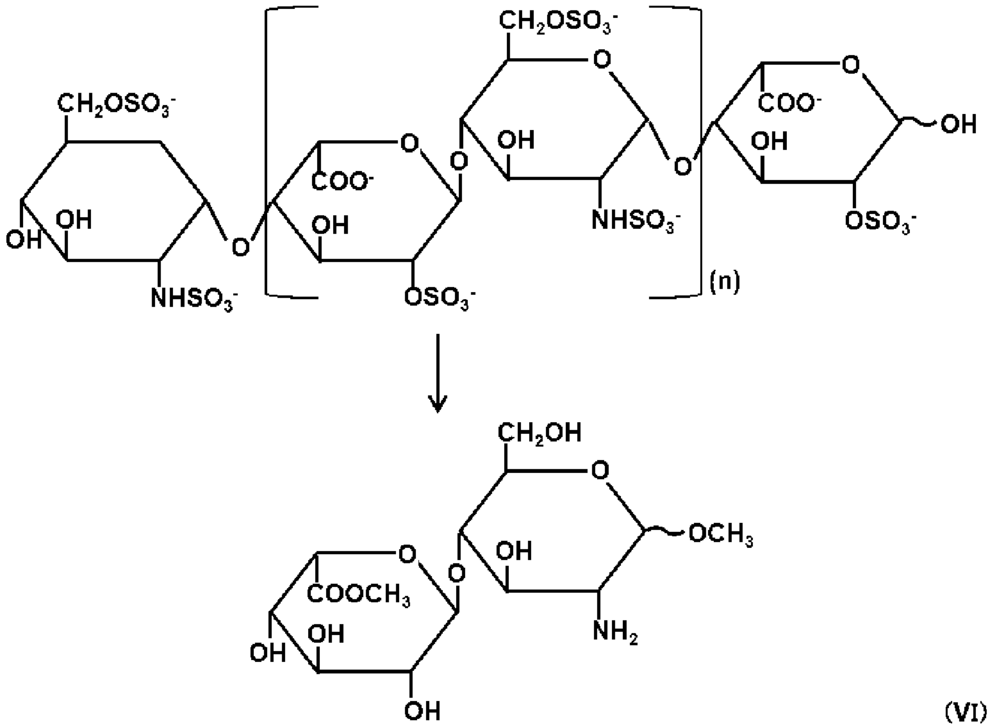

(VI)

[In Formula (VI), n represents an integer of 1 or more]

Methanolysis of dermatan sulfate is carried out by heating a sample containing dermatan sulfate in hydrogen chloride methanol solution containing 2,2-dimethoxypropane. Here, hydrogen chloride methanol solution refers to methanol containing hydrogen chloride. The concentration of hydrogen chloride contained in hydrogen chloride methanol solution is preferably 0.5 to 5mol/L, more preferably 1 to 4 mol/L, still more preferably 2.5 to 3.5 mol/L, for example

3mol/L. Here, the ratio of 2,2-dimethoxypropane to hydrogen chloride methanol solution is not particularly limited, but is, for example, 0.5 to 1.5:10, and particularly 1:10. The heating condition of the methanolysis reaction is preferably 20 minutes to 100 minutes at a temperature of 60°C to 80°C, more preferably 20 minutes to 90 minutes at a temperature of 60°C to 70°C, still more preferably 30 minutes to 80 minutes at a temperature of 63°C to 67°C, or 60 minutes to 80 minutes, for example, 75 minutes at a temperature of 65°C.

Methanolysis of heparan sulfate is performed by heating a sample containing heparan sulfate in hydrogen chloride methanol solution containing 2,2-dimethoxypropane. Here, the concentration of hydrogen chloride contained in hydrogen chloride methanol solution is preferably 0.5 to 5mol/L, more preferably 1 to 4mol/L, still more preferably 2.5 to 3.5mol/L, for example 3mol/L. Here, the ratio of 2,2-dimethoxypropane to hydrogen chloride methanol solution is not particularly limited, but is, for example, 0.5 to 1.5:10, and particularly 1:10. The heating condition of the methanolysis reaction is preferably from 80 minutes to 180 minutes at a temperature of from 65°C to 85°C, more preferably from 90 minutes to 180 minutes at a temperature of from 75°C to 85°C, still more preferably from 110 minutes to 130 minutes at a temperature of from 78°C to 82°C, for example, 120 minutes at a temperature of 80°C.

According to the method of the present invention, the amounts of dermatan sulfate and heparan sulfate contained in the sample can be measured. At this time, the sample to be measured is divided, one of which is used as a sample for measuring dermatan sulfate, and the other of which is used as a sample for measuring heparan sulfate. The sample used to measure the dermatan sulfate is heated under the conditions of the methanolysis reaction of the dermatan sulfate described above. This reaction decomposes the dermatan sulfate contained in the sample to a disaccharide. The sample used for measuring heparan sulfate is heated under the conditions of the methanolysis reaction of heparan sulfate described above. By this reaction, heparan sulfate contained in the sample is decomposed to disaccharide. At this time, the methanolysis reaction can be performed only on one of dermatan sulfate and heparan sulfate, and only one of them can be measured.

The disaccharide obtained by decomposing dermatan sulfate and/or heparan sulfate by methanolysis is subjected to liquid chromatography. The eluate from the liquid chromatography is then sequentially subjected to mass spectrometry.

The liquid chromatography used at this time is not particularly limited as long as the disaccharide represented by the above chemical formulae (I) to (IV) can be separated from other substances by once adsorbing the disaccharide on a column and then eluting the disaccharide.

For example, high performance liquid column chromatography using a column packed with a carrier capable of adsorbing disaccharides by ionic interaction, hydrophobic interaction, hydrophilic interaction, or the like can be suitably used in the method of the present invention.

The disaccharide produced by the methanolysis reaction exemplified by the above chemical reaction formulae (V) and (VI) has high polarity. Therefore, as a carrier which can be eluted after they are adsorbed once, a carrier which can retain disaccharide by hydrophilic interaction is particularly preferable. That is, high performance liquid chromatography using a carrier capable of retaining disaccharides by hydrophilic interaction is suitable as a method for separating disaccharides obtained by a methanolysis reaction. Such high performance liquid chromatography includes the hydrophilic interaction ultra-high performance liquid chromatography used in Example 4 below.

The flow path from the outlet of the liquid chromatography is connected to a mass spectrometer, and the eluate from the liquid chromatography is sequentially fed to the mass spectrometer.

There is no particular limitation on a mass spectrometer which can be used at this time. For example, the mass spectrometer may employ any ionization method including an atmospheric pressure photoionization method (APPI), an electron ionization method (EI), a chemical ion method, an electrodesorption method, a fast atomic bombardment method (FAB), a matrix-assisted laser desorption ionization method (MALDI), and an electrospray ionization method (ESI) as an ion source for ionizing molecules to be analyzed. In the mass spectrometer, the analyzer for separating ionized molecules may be of any type including a magnetic field deflection type, a quadrupole type, an ion trap type, and a tandem quadrupole type.

A mass spectrometer having an ion source operated by electrospray ionization (ESI) operating in a positive ion mode and a tandem quadrupole type analyzer can be suitably used in the method of the present invention. The tandem quadrupole mass spectrometer is a mass spectrometer in which a quadrupole (Q1) functioning as a mass filter, a quadrupole (Q2) functioning as a collision cell, and a quadrupole (Q3) functioning as a mass filter are arranged in series. In the quadrupole (Q1), target precursor ions are separated from a plurality of ions generated by ionization based on the mass-to-charge ratio (m/z) of the ions. The precursor ions are then collided with an inert gas or the like (e.g., argon) in the collision cell (Q2) to generate product ions (fragment ions). Next, in the quadrupole (Q3), the obtained product ions are selectively detected based on the mass-to-charge ratio (m/z).

Specific examples of methods for generating precursor ions and product ions from disaccharides obtained by methanolysis of dermatan sulfate represented by the above formula (V) by a tandem quadrupole mass spectrometer will be described below. First, by ionizing the disaccharide, precursor ions having a mass-to-charge ratio (m/z) of 426 are obtained. The precursor ion is represented by the following formula (VII).

[Chem. 7]

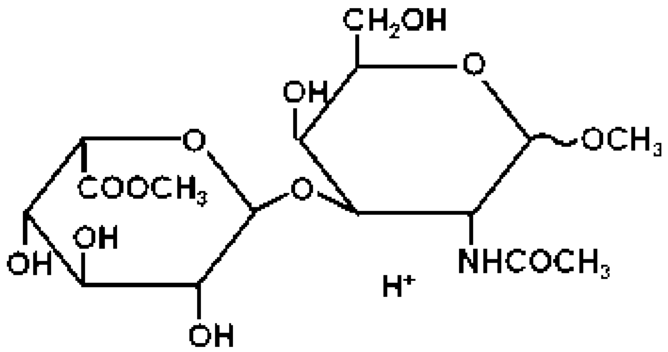

(VII)

A product ion having a mass-to-charge ratio (m/z) of 236 is obtained by separating the precursor ion represented by the above formula (VII) and cleaving the precursor ion in the collision cell (Q2). The cleavage of the precursor ion to obtain the product ion is shown in VIII.

[Chem. 8]

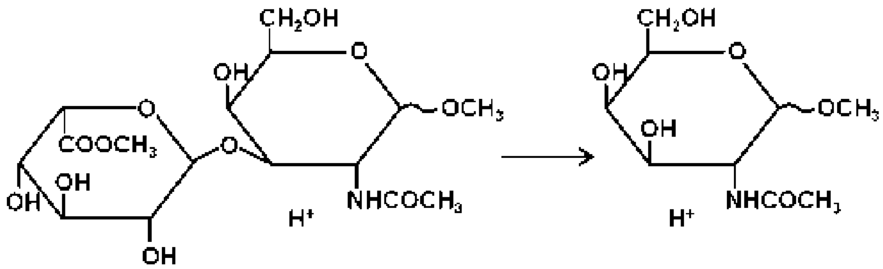

(VIII)

A specific example of a method of generating precursor ions and product ions from disaccharides obtained by methanolysis of heparan sulfate represented by the above formula (VI) by a tandem quadrupole mass spectrometer will be described below. First, by ionizing the disaccharide, precursor ions having a mass-to-charge ratio (m/z) of 384 are obtained. The precursor ion is represented by the following formula (IX).

[Chem. 9]

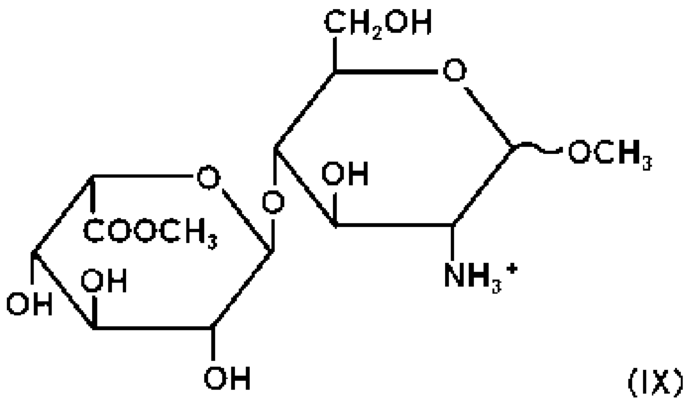

(IX)

The precursor ions represented by the above formula (IX) are separated and cleaved in the collision cell (Q2), whereby product ions having a mass-to-charge ratio (m/z) of 162 are obtained. The reaction in which the precursor ion is cleaved to obtain the product ion is represented by the general formula (X).

[Chem. 10]

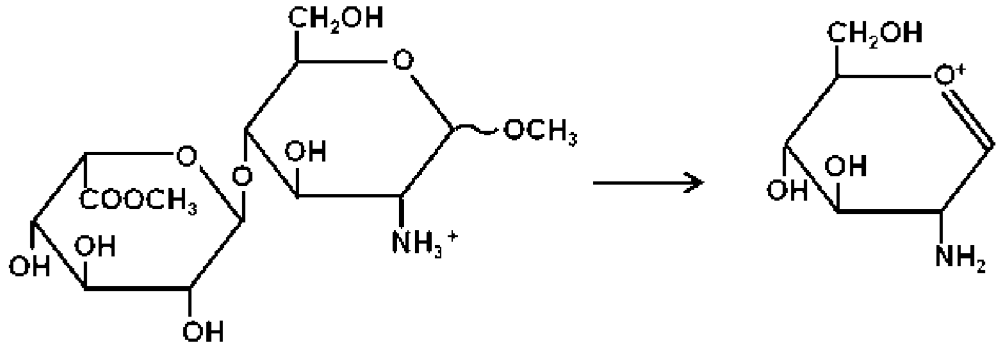

(X)

The amount of dermatan sulfate contained in the sample can be measured by the method using the liquid chromatography and the tandem quadrupole mass spectrometer described above. A known amount of dermatan sulfate is subjected to methanolysis and analyzed using a tandem quadrupole mass spectrometer to calculate an area of a peak (detection peak) detected on a chromatographic chart corresponding to a product ion having a mass-to-charge ratio (m/z) of 236. Then, a calibration curve showing the correlation between the amount of dermatan sulfate and the area of the detection peak is generated. A sample whose content of dermatan sulfate is unknown is subjected to methanolysis in the same manner and analyzed using a tandem quadrupole mass spectrometer to calculate the area of the detection peak corresponding to the product ion. By interpolating the obtained values into the calibration curve, the dermatan sulfate contained in the sample can be measured.

In the same manner, the amount of heparan sulfate contained in the sample can be measured. A known amount of heparan sulfate is subjected to methanolysis and analyzed using a tandem quadrupole mass spectrometer to calculate an area of a detection peak corresponding to product ions having a mass-to-charge ratio (m/z) of 162. Then, a calibration curve showing the correlation between the amount of heparan sulfate and the area of the detection peak is prepared. Also, a sample whose content of heparan sulfate is unknown is subjected to methanolysis in the same manner and analyzed using a tandem quadrupole mass spectrometer to calculate the area of the detection peak corresponding to the product ion. By interpolating the obtained value into the calibration curve, heparan sulfate contained in the sample can be measured.

The amount of dermatan sulfate and heparan sulfate contained in the sample can also be measured. In this case, the sample to be measured is divided, one of which is used as a sample for measuring dermatan sulfate, and the other of which is used as a sample for measuring heparan sulfate. The sample used to measure the dermatan sulfate is heated under the conditions of the methanolysis reaction of the dermatan sulfate described above. The sample used for measuring heparan sulfate is heated under the conditions of the methanolysis reaction of heparan sulfate described above. Methanolysis decomposition products of dermatan sulfate and heparan sulfate, respectively, are subjected to liquid chromatography to obtain an eluate, and the eluate is sequentially analyzed by a mass spectrometer, whereby the amounts of dermatan sulfate and heparan sulfate contained in the sample can be measured.

In the present invention, samples to be analyzed include, but are not limited to, for example, body fluids, cells, tissues, organs, cell cultures, tissue cultures, foods, and feedstuffs, or those derived therefrom.

When the sample is a cell, there is no particular limitation on the species from which the cell is derived. The species may be prokaryotic or eukaryotic, e.g., bacteria, yeast, plants, birds, amphibians, reptiles, mammals. When the species is a mammal, the animal species is not particularly limited, for example, humans, monkeys, mice, rats, guinea pigs, hamsters, rabbits, horses, cows, pigs, dogs, cats, particularly humans.

When the sample is a bodily fluid, tissue or organ, there is no particular limitation on the species from which these are derived. The species include, for example, plants, birds, amphibians, reptiles, mammals, particularly mammals. When the species is a mammal, the animal species is not particularly limited, for example, humans, monkeys, mice, rats, guinea pigs, hamsters, rabbits, horses, cows, pigs, dogs, cats, particularly humans.

When the sample is derived from a mammal, the sample may be, for example, a bodily fluid, blood, serum, plasma, bone marrow fluid, cerebrospinal fluid, urine, or derived therefrom obtained from the mammal. Here, the body fluid refers to all of the humoral components derived from mammals, including blood, bone marrow fluid, cerebrospinal fluid, saliva, tears, sweat, semen, synovial fluid, and urine. Serum or plasma can be prepared from blood by known methods such as centrifugation.

Also, if the sample is derived from a mammal, the sample may be, for example, a cell, tissue, organ, or derived therefrom obtained from the mammal. Here, the type of the cell is not particularly limited, but examples of the cell include

mesenchymal stem cells, neural cells, neuroblasts, myoblasts, glial cells, Schwann cells, cardiomyocytes, skeletal muscle cells, smooth muscle cells, chondrocytes, osteoblasts, fibroblasts, keratinocytes, epithelial cells, endothelial cells, corneal epithelial cells, corneal endothelial cells, retinal cells, hepatocytes, mesangial cells, mesenchymal cells, hematopoietic cells, dendritic cells, and stromal cells. The tissue is not particularly limited, and examples of the tissue include epithelial tissue, connective tissue, muscle tissue, nerve tissue, fibrous connective tissue, cartilage tissue, and bone tissue. The organ is not particularly limited, and examples of the organ include stomach, small intestine, large intestine, liver, pancreas, kidney, spleen, heart, lung, pituitary gland, testis, ovary, cerebellum, cerebrum, diencephalon, midbrain, medulla oblongata, and pituitary gland.

Diseases in which dermatan sulfate and heparan sulfate accumulate in the body are known. Such diseases include, for example, Hunter syndrome, Hurler syndrome, Scheie syndrome, Hurler-Scheie syndrome, and Maroteaux-Lamy syndrome. In such diseases, therapies have been attempted to relieve symptoms by decreasing dermatan sulfate and heparan sulfate abnormally accumulated in the body. Enzyme replacement therapy, in which enzymes that break down dermatan sulfate and heparan sulfate are administered into the body, is an example of such therapies.

Patients with diseases in which dermatan sulfate and heparan sulfate accumulate in the body can be screened based on measurements of dermatan sulfate and heparan sulfate accumulated in the body. If the measured value is abnormally higher than a normal human, the subject can be considered to be a patient with the disease. The method of the present invention may be practiced for the purpose of making such determinations.

The effectiveness of the treatment in treating patients with diseases in which dermatan sulfate and heparan sulfate accumulate in the body can be confirmed by measuring the dermatan sulfate and heparan sulfate accumulated in the patient's body before and after the treatment and measuring the amount of these reductions after the treatment. The larger the amount of reduction, the higher the therapeutic effect can be judged. The method of the present invention can be carried out for the purpose of confirming such therapeutic effect. For example, a therapeutic effect can be determined if the dermatan sulfate and heparan sulfate accumulated in the patient's body are each reduced before and after treatment, preferably by 10% or more, more preferably by 20% or more, still more preferably by 30% or more, and even more preferably by 50% or more.

In addition, screening of drugs for the treatment of patients with diseases in which dermatan sulfate and heparan sulfate accumulate in the body can be performed by administering the test drug to the experimental animals, measuring the dermatan sulfate and heparan sulfate accumulated in the body of the experimental animals before and after administration of the test drug, and measuring the amount of decrease of these after administration. The greater these reductions, the greater the therapeutic effect of the test drug. For example, if the dermatan sulfate and heparan sulfate accumulated in the patient's body decrease before and after treatment, respectively, preferably by 10% or more, more preferably by 20% or more, still more preferably by 30% or more, and even more preferably by 50% or more, the therapeutic effect of the test agent can be considered to be high.

Hunter syndrome is a disease that genetically lacks some or all of iduronic acid-2-sulfatase activity. Deficiency or deletion of iduronic acid-2-sulfatase results in accumulation of dermatan sulfate and heparan sulfate in the patient's body. Thus, the methods of the present invention can be used to screen patients with Hunter syndrome, to confirm the efficacy of the therapy, to evaluate the efficacy of the therapeutic agent, to screen for the test agent, and the like. Drugs for treating Hunter syndrome include, but are not limited to, human iduronate-2-sulfatase, analogs of human iduronate-2-sulfatase, and conjugates of antibodies to human iduronate-2-sulfatase. Conjugates of human iduronic acid-2-sulfatase and antibodies include, for example, fusion proteins of human iduronic acid-2-sulfatase and anti-human transferrin receptor antibodies.

Hurler syndrome, Scheie syndrome, and Hurler-Scheie syndrome are diseases that genetically lack some or all of L-iduronidase activity. Deficiency or deletion of L-iduronidase results in accumulation of dermatan sulfate and heparan sulfate in the patient's body. Thus, the methods of the present invention can be used to screen patients, confirm the efficacy of therapeutics, evaluate the efficacy of therapeutic agents, screen test agents, and the like for these diseases. Therapeutic agents for these diseases include, but are not limited to, L-iduronidase, an analogue of L-iduronidase, a conjugate of L-iduronidase and an antibody. Conjugates of L-iduronidase and antibodies include, for example, fusion proteins of L-iduronidase and anti-human transferrin receptor antibodies.

Maroteaux-Lamy syndrome is a disease that genetically lacks some or all of N-acetylgalactosamine-4-sulfatase (ASB) activity. Deficiency or deletion of ASB results in the accumulation of dermatan sulfate, in particular, in the patient's body. Thus, the methods of the present invention can be used to screen patients with Maroteaux-Lamy syndrome, to confirm the efficacy of the therapy, to evaluate the efficacy of the therapeutic agent, to screen for the test agent, and the like. Drugs for the treatment of Maroteaux-Lamy syndrome include, but are not limited to, ASB and analogs of ASB.

Sanfilippo syndrome is a disease that is classified into types A, B, C, and D, and genetically lacks some or all of heparan sulfate N-sulfatase activity, $\alpha$-N-acetylglucosaminidase activity, acetyl CoA: $\alpha$-glucosaminide N-acetyltransferase activity, and N-acetylglucosamine-6-sulfate sulfatase activity, respectively. The deficiency or deletion of these enzymes results in the accumulation of heparan sulfate, in particular, in the body of the patient. Thus, the methods of the present invention can be used to screen patients with Sanfilippo syndrome, to confirm the efficacy of the therapy,

to evaluate the efficacy of the therapeutic agent, to screen for the test agent, and the like. Drugs for the treatment of Sanfilippo syndrome include, but are not limited to, these enzymes, analogs of these enzymes, and conjugates of antibodies with any of these enzymes. Conjugates of these enzymes and antibodies include, for example, fusion proteins of these enzymes and anti-human transferrin receptor antibodies.

Sly syndrome is a disease that genetically lacks some or all of β-glucuronidase. Deficiency or deletion of β-glucuronidase results in accumulation of dermatan sulfate and heparan sulfate in the patient's body. Thus, the methods of the present invention can be used to screen patients with Sly syndrome, to confirm the efficacy of the therapy, to evaluate the efficacy of the therapeutic agent, to screen for the test agent, and the like. Drugs for the treatment of Sly syndrome include, but are not limited to, β-glucuronidase, an analog of β-glucuronidase, and a conjugate of β-glucuronidase and an antibody. Conjugates of β-glucuronidase and antibodies include, for example, fusion proteins of β-glucuronidase and anti-human transferrin receptor antibodies.

The method of the present invention can also be used as a method for determining the amount of heparan sulfate or/and dermatan sulfate contained in food and feedstuff. For example, the intake of heparan sulfate or/and dermatan sulfate may be controlled by pre-measuring the amount of heparan sulfate or/and dermatan sulfate contained in food or feedstuff ingested by humans or non-human animals. When a patient with the above-mentioned disease is in a state in which the intake of heparan sulfate or the like is to be limited, the intake of heparan sulfate or the like can be appropriately controlled by consuming a food in which the amount of heparan sulfate or the like has been measured in advance by the method of the present invention.

[EXAMPLES]

The invention will now be described in more detail with reference to examples, which are not intended to be limiting.

[Example 1] Preparation of each solution

Solutions of (a) to (l) used in the test were prepared by the following procedure.

(a) MeCN/water:

0.5 mL of water for injection and 4.5 mL of acetonitrile were mixed to make a MeCN/water. This solution was prepared before use.

(b) Deuterium labeled solvent:

In an ice bath, 240 μL of acetyl chloride was added dropwise to 1.5 mL of methanol-$d_4$ (Sigma-Aldrich Inc.) as a deuterium-labeled solvent. This solution was prepared before use.

(c) PBS/citric acid solution:

Citric acid was dissolved in pure water to prepare a citric acid solution at a concentration of 10 mM. Furthermore, trisodium citrate dihydrate was dissolved in pure water to prepare a sodium citrate solution having a concentration of 10 mM. Sodium citrate solution was added dropwise to the citric acid solution to adjust to pH 3.0. The solution was 10 mM citrate buffer, pH 3.0. A solution obtained by adding 2 mL of PBS to 18 mL of 10 mM citrate buffer (pH 3.0) was used as a PBS/citric acid solution.

(d) Mobile phase A:

To 247.5 mL of pure water was added 2.5 mL of a 1 M aqueous solution of ammonium formate and 400 μL of an aqueous solution of ammonium hydroxide (25% $NH_4OH$) and mixed to form a mobile phase A. This solution was prepared before use.

(e) Mobile phase B:

A mixture of 45 mL of pure water and 5 mL of 1 M ammonium formate aqueous solution, 450 mL of acetonitrile, and 800 μL ammonium hydroxide aqueous solution (25% $NH_4OH$) was used as a mobile phase B. This solution was prepared before use.

Redissolving solution

(f) Heparan Sulfate Standard Stock Solution (HS Standard Stock Solution):

Heparan sulfate (Iduron Inc.) was weighed in a 1.5 mL micro tube and dissolved by adding water for injection to prepare a 5.0 mg/mL solution. The prepared solution was dispensed into 0.5 mL screw cap tubes in 15 μL portions and stored frozen (-15°C or less) until use. This solution was used as the HS standard stock solution.

(g) Dermatan Sulfate Standard Stock Solution (DS Standard Stock Solution):

Chondroitin sulfate B sodium salt from porcine intestinal mucosa (Sigma-Aldrich Inc.) was weighed in a 1.5 mL micro tube and dissolved by adding water for injection to prepare a 5.0 mg/mL solution. The prepared solution was dispensed into 0.5 mL screw cap tubes in 15 μL portions and stored frozen (-15°C or less) until use. This solution was used as the DS standard stock solution.

(h) Dermatan Sulfate Internal Standard Solution (DS Internal Standard Solution):

40 μL of the DS standard stock solution was measured out in a borosilicate screw-top test tube, and the solvent was distilled off under a stream of nitrogen. Deuteriomethanolysis was carried out by adding 400 μL of deuterium-labeled solvent to the dry matter, agitating the solvent, and reacting the solvent at 65°C for 75 minutes. After the reaction, the solvent was distilled off under a stream of nitrogen. To the dry matter was added 500 μL of MeCN/water and sonicated for 30 minutes. The prepared solution was dispensed into 0.5 mL screw cap tubes in 20 μL portions and stored frozen (-15°C or less). This solution was used as an internal standard solution of dermatan sulfate (DS internal standard solution).

(i) Heparan Sulfate Internal Standard Solution (HS Internal Standard Solution):

40 μL of HS standard stock solution was measured out in a borosilicate screw-top test tube, and the solvent was distilled off under a stream of nitrogen. Deuteriomethanolysis was carried out by adding 400 μL of deuterium-labeled solvent to the dry matter, agitating the solvent, and reacting the solvent at 65°C for 75 minutes. After the reaction, the solvent was distilled off under a stream of nitrogen. To the dry matter was added 500 μL of MeCN/water and sonicated for 30 minutes. The prepared solution was dispensed into 0.5 mL screw cap tubes in 20 μL portions and stored frozen (-15°C. or less). This solution was used as an internal standard solution of heparan sulfate (HS internal standard solution).

(j) Solution for sample dissolution:

To a 5-mL MeCN/water, 1 μL of HS internal standard solution and 1 μL of DS internal standard solution were added and stirred, followed by sonication for 30 minutes. This solution was used as a solution for sample dissolution. This solution was prepared before use.

(k) Solution for calibration graph:

480 μL of PBS/citric acid solution was measured out, and 10 μL each of HS standard stock solution and DS standard stock solution was added thereto to prepare a solution containing 100 μg/mL each of dermatan sulfate and heparan sulfate. This solution was diluted with a PBS/citric acid solution to prepare a solution containing 2500 ng/mL each of dermatan sulfate and heparan sulfate. This solution was diluted in two steps with PBS/citric acid solution to prepare a solution containing dermatan sulfate and heparan sulfate at concentrations of 25 to 2500 ng/mL, respectively. This solution was used as a solution for preparing a calibration curve.

(l) Tissue extraction solution

Physiological saline was added to 1 mL of polyoxyethylene(10)octylphenyl ether to make a total volume of 500 mL. This solution was used as a tissue extraction solution.

[Example 2] Preparation of blood samples

Blood was collected from wild-type mice (C57BL/6N) and hemizygous mice lacking chromosomal regions containing IDS genes (IDS hemizygous mice, C57BL/6N). The collected blood was transferred to a tube and left to stand at room temperature for 30 minutes or longer, and then centrifuged (2000 g, 20 minutes) to collect serum. To 8 μL of

sera, 8 μL of PBS and 144 μL of 10mM citrate buffer (pH 3.0) were added and stirred. This was measured out in 20 μL and separated into borosilicate screw-top test tubes. This was used as a blood sample solution.

[Example 3] Methanolysis reaction

Each of the calibration curve preparation solutions prepared in Example 1 above was measured out in 20 μL and individually separated into borosilicate screw-top test tubes. Also, as blanks, PBS/citric acid solutions were separated into borosilicate screw-top test tubes. The solvent in the tube was distilled off under a stream of nitrogen (N=1). To the dried product was added 20 μL of 2,2-dimethoxypropane and 200 μL of hydrogen chloride methanol solution having 3N concentrations of hydrochloric acid, followed by agitation, followed by methanolysis reactions under three conditions (Conditions A to C) shown in Table 1. After the reaction, the solvent was distilled off under a stream of nitrogen. After 50 μL of the sample dissolving solution was added to the dried matter and dissolved, the solution was centrifuged (15000 rpm, room temperature for 10 minutes) and the supernatant was collected in a vial.

In addition, the dermatan sulfate standard stock solution and the heparan sulfate standard stock solution prepared in Example 1 above were diluted with PBS/citric acid solution, and four quality control samples (QC samples) containing both the dermatan sulfate standard stock solution and the heparan sulfate standard stock solution at concentrations of 25.0 ng/mL, 50.0 ng/mL, 500 ng/mL, and 2000 ng/mL were prepared and used as QC-LL,QC-L,QC-M and QC-H, respectively. Each of these samples was measured out to 20 pL, and separated into borosilicate screw-top test tubes, and the solvent in the test tubes was distilled off under a stream of nitrogen. To the dried product was added 20 μL of 2,2-dimethoxypropane and 200 μL of hydrogen chloride methanol solution having 3N concentrations of hydrochloric acid, followed by agitation, followed by methanolysis reactions under three conditions (Conditions A to C) shown in Table 1. After the reaction, the solvent was distilled off under a stream of nitrogen. After 50 μL of the sample dissolving solution was added to the dried matter and dissolved, the solution was centrifuged (15000 rpm, room temperature for 10 minutes) and the supernatant was collected in a vial.

In addition, 20 μL of the blood sample solution prepared in Example 2 was measured out, separated into borosilicate screw-top test tubes, and the solvent in the test tubes was distilled off under a stream of nitrogen. To the dried product was added 20 μL of 2,2-dimethoxypropane and 200 μL of hydrogen chloride methanol solution having 3N concentrations of hydrochloric acid, followed by agitation, followed by methanolysis reactions under three conditions (Conditions A to C) shown in Table 1. After the reaction, the solvent was distilled off under a stream of nitrogen. After 50 μL of the sample dissolving solution was added to the dried matter and dissolved, the solution was centrifuged (15000 rpm, room temperature for 10 minutes) and the supernatant was collected in a vial.

[Table 1]

Table 1 Conditions for methanolysis reaction

| | Heating Temperature (°C) | Heating Time |
|---|---|---|
| Condition A | 65 | 75 minutes |
| Condition B | 80 | 2 hours |
| Condition C | 65 | 18 hours |

[Example 4] LC/MS/MS spectrometry

LC/MS/MS spectrometry was performed using a combination of hydrophilic interactive ultrahigh performance liquid chromatography and tandem quadrupole mass spectrometry. QTRAP5500 (AB Sciex Inc.) was used as a mass spectrometer (MS/MS device), and Nexera X2 (Shimadzu Seisaksuho) was set as a HPLC device. Acquity UPLC™ BEH Amid 1.7μm (2.1X50 mm, Waters Inc.) was used as the LC-column. Mobile phases A and B prepared in Example 1 were used as mobile phases. The column temperature was set at 50°C.

After equilibrating the column with a mixture of 6% (v/v) mobile phase A and 94% (v/v) mobile phase B, 10μL of sample was injected and chromatographed under the mobile phase gradient conditions shown in Table 2. The flow rate of the mobile phase was 0.4 mL/min.

[Table 2]

Table 2 Conditions of liquid chromatography

| Elapsed time after sample injection (min) | Mobile phase A (%(v/v)) | Mobile phase B (%(v/v)) |
|---|---|---|
| 0 | 6 | 94 |
| 1 | 6 | 94 |
| 4 | 30 | 70 |
| 4.01 | 6 | 94 |
| 6 | 6 | 94 |

The ion source parameters of the MS/MS device were set as shown in Table 3 according to QTRAP5500's instructions.

[Table 3]

Table 3 Setting parameters of the ion source of the MS / MS instrument

| | |
|---|---|
| Ion Source | ESI (TurboV) |
| Polarity | Positive |
| Scan type | MRM |
| IonSpray voltage | 5500 V |
| Heater gas temperature | 500° C |
| Curtain gas (CUR) | 30.0 psi |
| Col lisi on gas (CAD) | 8 psi |
| Ion Source Gas 1 (GS1) | 70.0 psi |
| Ion Source Gas 2 (GS2) | 70.0 psi |
| Entrance Potential | 10.0 V |
| Duration | 4.00 min |

The areas of the peaks (detection peaks) detected on the chromatograph charts of the product ions derived from dermatan sulfate and heparan sulfate contained in each calibration curve preparation solution were determined by measurement of the calibration curve preparation solution and QC samples. The areas of the detection peaks of the product ions derived from the DS internal standard solution and the HS internal standard solution were also determined. Calibration curve preparation solutions were measured at N=1, and QC samples (QC-LL,QC-L,QC-M and QC-H) were measured at N=3.

The product ions detected here are ions obtained by the above reaction formula (VIII) for dermatan sulfate and ions obtained by the above reaction formula (X) for heparan sulfate. The dermatan sulfate and heparan sulfate contained in the DS internal standard solution and the HS internal standard solution are labeled with deuterium. Therefore, the mass-to-charge ratio (m/z) of the precursor ions derived therefrom takes a large value (432 and 390, respectively) compared to that of the unlabeled ones. The mass to charge ratio (m/z) of precursor ions from unlabeled dermatan sulfate and heparan sulfate are 426 and 384, respectively. Therefore, these precursor ions are separated at the quadrupole (Q1) based on the mass-to-charge ratio (m/z) of the ions, so that they can be detected individually. Table 4 summarizes the m/z of precursor and product ions.

Ion chromatograms obtained by LC/MS/MS spectrometry of solutions for preparing calibration curves containing 25 ng/mL each of dermatan sulfate and heparan sulfate, which have been subjected to methanolysis under the condition A shown in Table 1, are shown in Figure 1. Figure 1 shows the results of sequential detection of the amount of ions

contained in the eluate from liquid chromatography at m/z=426 corresponding to precursor ions of dermatan sulfate. In the figure, the black peak corresponds to the precursor ion of dermatan sulfate.

Figure 2 shows an ion chromatogram, obtained by LC/MS/MS spectrometry, of a solution for preparing a calibration curve containing 25 ng/mL each of dermatan sulfate and heparan sulfate, the solution being subjected to a methanolysis treatment under the condition B shown in Table 1. Figure 2 shows the results of sequentially detecting the amount of ions contained in the eluate from liquid chromatography at m/z=384 corresponding to precursor ions of heparan sulfate. In the figure, a black peak corresponds to a precursor ion of heparan sulfate.

[Table 4]

Table 4 (m/z) values of precursor ion and product ion

| | Precursorion (m/z) | Production (m/z) |
|---|---|---|
| Dermatan sulfate | 426 | 236 |
| Dermatan sulfate (deuterium-labeled) | 432 | 239 |
| Heparan sulfate | 384 | 162 |
| Heparan sulfate (deuterium-labeled) | 390 | 162 |

The area ratio (DS detection peak area/ DS-IS detection peak area) of the detection peak (DS-IS detection peak area) from the DS internal standard solution to the area of the detection peak (DS detection peak area) from dermatan sulfate contained in the solutions for preparing the calibration curves was determined. This value is taken as the vertical axis, and the concentration of delmatan sulfate in each solution for preparation as the horizontal axis. The regression formula was calculated using the quadratic programming method to create a test line.

The area ratio (HS detection peak area/HS-IS detection peak area) of the detection peak (HS-IS detection peak area) from the HS internal standard solution to the area of the detection peak (HS detection peak area) from heparan sulfate contained in the solutions for preparing the calibration curves was determined. This value is taken as the vertical axis, and the concentration of Heparan sulfate in each test solution is taken as the horizontal axis. The regression formula was calculated using the quadratic programming method to create a test line.

Further, the precision (%) and the trueness (%) were obtained by the following calculation formula on the basis of the measured value of the QC sample.

Precision (%) = Standard deviation of measured values/Mean of measured values × 100

Trueness (%) = Mean measured value/theoretical concentration × 100

The theoretical concentration in the calculation formula of the trueness (%) refers to the concentration of dermatan sulfate or heparan sulfate added to the QC sample.

[Example 5] Examination of the calibration curve obtained under condition A

Calibration curves (calibration curves of Condition A) obtained from the measured values of the solutions for preparation of the calibration curves that were subjected to methanolysis under Condition A shown in Table 1 above showed good linearity in the concentrations ranging from 25.0 to 2500 ng/mL for both dermatan sulfate and heparan sulfate (data not shown). The correlation coefficient (r) was 0.9995 for dermatan sulfate and 0.9938 for heparan sulfate.

Next, the precision and trueness will be examined. Tables 5 and 6 show the precision and trueness of the measurements for each QC sample. These values indicate that condition A is suitable as a methanolysis condition for measuring concentrations of dermatan sulfate and heparan sulfate ranging from 25.0 to 2500 ng/mL. However, measurements of heparan sulfate at concentrations of 25.0 ng/mL were significantly reduced in both precision and trueness (Table 5). Dermatan sulfate, on the other hand, did not significantly reduce the precision and trueness of measurements at concentrations of 25.0 ng/mL (Table 6).

[Table 5]

Table 5 Precision and trueness of measurements of heparan sulfate in QC samples

| QC sample | Concentration of heparan sulfate (ng/mL) | Precision (%) | Trueness (%) |
|---|---|---|---|
| QC-LL | 25.0 | 27.8 | 65.9 |
| QC-L | 50.0 | 10.2 | 57.4 |
| QC-M | 500 | 9.7 | 102.4 |
| QC-H | 2000 | 6.1 | 95.1 |

[Table 6]

Table 6 Precision and trueness of measurements of dermatan sulfate in QC samples

| QC sample | Concentration of dermatan sulfate (ng/mL) | Precision (%) | Trueness (%) |
|---|---|---|---|
| QC-LL | 25.0 | 13.6 | 104.1 |
| QC-L | 50.0 | 6.2 | 88.4 |
| QC-M | 500 | 4.6 | 98.5 |
| QC-H | 2000 | 3.8 | 98.4 |

[Example 6] Comparison of calibration curves obtained under conditions A, B and C

The calibration curves obtained from the measured values of the solutions for preparation of the calibration curves, which were subjected to methanolysis under conditions A, B and C, showed good linearity at concentrations ranging from 25 to 2500 ng/mL for both dermatan sulfate and heparan sulfate (data not shown). The correlation coefficients (r) of the calibration curves under the respective conditions are shown in Table 7.

[Table 7]

Table 7 Correlation coefficient (r) of calibration curve under conditions A, B and C

| | Correlation coefficient (r) | |
|---|---|---|
| | Dermatan sulfate | Heparan sulfate |
| Condition A | 0.9995 | 0.9938 |
| Condition B | 0.9970 | 0.9993 |
| Condition C | 0.9972 | 0.9999 |

However, in Example 5, condition A reduced both the precision and trueness of heparan sulfate measurements at concentrations of 25.0 ng/mL. Therefore, a condition for more accurately measuring heparan sulfate concentrations of 25.0 ng/mL was investigated. Therefore, the precision and trueness of heparan sulfate measurements of QC samples (QC-LL) under conditions A, B and C were compared. The QC sample (QC-LL) contains dermatan sulfate and heparan sulfate at concentrations of 25.0 ng/mL, respectively. The results are shown in Table 8. With heparan sulfate, measured values with high precision and trueness were obtained when measured under condition B. In the case of heparan sulfate, even when measured under the condition C, measured values having high precision and trueness are obtained, but the condition C has a disadvantage that a long time is required for heat treatment. Therefore, it was concluded that the methanolysis for the measurement of heparan sulfate is favorable under condition B. In the same manner as in Example 5, the measured value of heparan sulfate decreased both in precision and trueness under the condition A.

For dermatan sulfate, the precision and trueness of the measurements under conditions A, B and C were also compared. The results are shown in Table 8. Dermatan sulfate reduced both precision and trueness in conditions B and C compared to condition A.

These results indicate that the methanolysis for the measurement of dermatan sulfate is favorable under condition A, and the methanolysis for the measurement of heparan sulfate is favorable under condition B.

[Table 8]

Table 8 Precision and trueness of measurements of dermatan sulfate and heparan sulfate in QC sample (QC-LL) under conditions A, B and C

| | Dermatan sulfate | | Heparan sulfate | |
|---|---|---|---|---|
| | Precision (%) | Trueness (%) | Precision (%) | Trueness (%) |
| Condition A | 1.0 | 101.2 | 9.1 | 125.1 |
| Condition B | 43.3 | 61.3 | 1.5 | 90.8 |
| Condition C | 24.1 | 121.9 | 0.2 | 105.7 |

[Example 7] Comparison of measurement results of dermatan sulfate and heparan sulfate contained in serum under conditions A, B and C

As a method for measuring dermatan sulfate and heparan sulfate contained in a sample derived from a living body, which of conditions A, B, and C is suitable was examined using sera prepared from blood of wild-type mice and IDS hemizygote mice described in Example 2. IDS is an enzyme having an activity of degrading GAG including dermatan sulfate and heparan sulfate. Thus, the concentration of GAG in the blood of IDS hemizygote mice is known to be higher compared to wild-type mice.

Measurement results of dermatan sulfate in sera collected from wild-type mice and IDS hemizygote mice under conditions A, B, and C are shown in Figure 3, and measurement results of heparan sulfate are shown in Figure 4.

Measurements of dermatan sulfate were higher in IDS hemizygote mice compared to wild-type mice under condition A (Figure 3) . This result reflects the genotype of IDS hemizygous mice. On the other hand, the measured values of dermatan sulfate in Conditions B and C were almost the same in wild-type mice and IDS hemizygote mice, and were extremely high compared with the measured results in Condition A (Figure 3). These results are considered to indicate that the product ions derived from components other than dermatan sulfate contained in the serum were generated under the conditions B and C. That is, in the conditions B and C, the measurement of dermatan sulfate in serum is difficult.

On the other hand, the measured value of heparan sulfate was higher in the IDS hemizygote mouse than in the wild-type mouse under any of the conditions A to C, and the measured value reflected the genotype of the IDS hemizygote mice (Figure 4). However, from the results shown in Table 8, it can be said that the measured value of heparan sulfate is higher in precision and trueness than the value measured under the condition B than the value measured under the condition A. Therefore, even in the measurement results of the conditions A to C shown in Figure 4, it can be said that the measured value under the condition B is higher in both precision and trueness than the measured value under the condition A.

From the above results, it can be concluded that the condition A (heating temperature: 65°C, heating time: 75 minutes) is preferable as the condition for the methanolysis of dermatan sulfate contained in the serum, and the condition B (heating temperature: 80°C, heating time: 2 hours) is preferable as the condition for the methanolysis of heparan sulfate.

[Example 8] Determination of dermatan sulfate and heparan sulfate contained in the blood of mice Administered with IDS

Recombinant human iduronic acid-2-sulfatase (rhIDS) commercially available for medical purposes was diluted with physiological saline to prepare 0.1 mg/mL of rhIDS solution. The rhIDS solutions were administered intravenously to hemizygous mice lacking chromosomal regions containing IDS genes (IDS hemizygous mice, C57BL/6N) at doses of 0.5 mg/kg body weight for a total of four times every seven days. Seven days after the last administration, mice were euthanized by exsanguination. At this time, blood was collected in a tube, left to stand at room temperature for 30 minutes or more, and then centrifuged (2000 g, 20 minutes) to collect serum. Similarly, sera were collected from IDS hemizygote mice without rhIDS (rhIDS non-treated IDS hemizygote mice) and wild-type mice.

The sera collected from the mice were agitated by adding 2 μL of serum, 8 μL of PBS, and 18 μL of 10 mM citrate buffer (pH 3.0) to two borosilicate screw-top tubes, respectively. This was used as a blood sample solution. The blood sample solution was subjected to a methanolysis reaction under the conditions B (heating temperature 80°C, heating time 2 hours) and A (heating temperature 65°C, heating time 75 minutes) described in Example 3. Next, the blood sample solution after the methanolysis was subjected to the LC/MS/MS spectrometry described in Example 4 to quantify dermatan sulfate and heparan sulfate contained in the blood sample solution. Here, the determination of dermatan sulfate was performed using a sample subjected to a methanolysis reaction under condition A, and the determination of heparan sulfate was performed using a sample subjected to a methanolysis reaction under condition B. Measurements were performed using 3 to 4 mice in both wild-type, IDS-treated and non-IDS-treated hemizygotes.

The quantitative results of heparan sulfate and dermatan sulfate in the blood sample solution are shown in Figures 5A and 5B, respectively. The quantitative values of heparan sulfate and dermatan sulfate were both higher in rhIDS non-IDS-hemizygote mice compared with wild-type mice. In addition, both the quantitative values of heparan sulfate and dermatan sulfate were decreased in rhIDS IDS hemizygote mice compared with rhIDS non-IDS hemizygote mice. These results indicate that the heparan sulfate and dermatan sulfate contained in the blood can be quantified by treating the blood sample solution with Condition B (heating temperature: 80°C, heating time: 2 hours) as a condition of methanolysis for quantifying heparan sulfate, Condition A (heating temperature: 65°C, heating time: 75 minutes) as a condition of methanolysis for quantifying dermatan sulfate. Furthermore, the obtained quantitative values indicate that the effect of enzyme replacement therapy using IDS or a substance having IDS activity can be quantitatively verified.

[INDUSTRIAL APPLICABILITY]

According to the present invention, since the concentration of a trace amount of heparan sulfate and/or dermatan sulfate contained in a sample can be accurately measured, it can be used for, for example, screening of patients with Hunter syndrome in which GAG such as heparan sulfate or dermatan sulfate accumulates in the body.

## Claims

1. A method of decomposing heparan sulfate contained in a sample to a disaccharide in which a uronic acid and an amino sugar are joined with an α-1,4 bond, wherein the heparan sulfate is decomposed by heating in hydrogen chloride methanol solution containing 2,2-dimethoxypropane, for 80 to 180 minutes, and at a temperature of 65 to 85°C.

2. The method according to claim 1, wherein the heating is performed for 110 to 130 minutes and at a temperature of 78 to 82°C.

3. The method according to claim 1 or 2, wherein the sample is selected from or derived from any one of a bodily fluid, a cell, a tissue, an organ, a cell culture, a tissue culture, a food, and a feedstuff.

4. The method according to claim 1 or 2, wherein the sample is selected from or derived from any one of a bodily fluid, a cell, a tissue, an organ, a blood, a serum, a plasma, a urine, a bone marrow fluid, and a cerebrospinal fluid, obtained from a mammal.

5. The method according to claim 4, wherein the mammal is selected from the group consisting of a human, a monkey, a mouse, a rat, a guinea pig, a hamster, a rabbit, a horse, a bovine, a pig, a dog, and a cat.

6. The method according to claim 4, wherein the mammal is a human, and the human is a patient with a disease in which the heparan sulfate accumulates in a body.

7. The method according to claim 6, wherein the disease is selected from the group consisting of Hunter syndrome, Hurler syndrome, Scheie syndrome, Hurler-Scheie syndrome, Sanfilippo syndrome, and Sly syndrome.

8. The method according to claim 6 or 7, wherein the patient has been treated to reduce the heparan sulfate accumulated in the body.

9. A method for decomposing dermatan sulfate and heparan sulfate contained in a sample into a disaccharide, respectively, wherein
the dermatan sulfate is decomposed into a disaccharide in which a uronic acid and an amino sugar are joined with

an α-1,3 bond, and the heparan sulfate is decomposed into the disaccharide in which the uronic acid and the amino sugar are joined with the α-1,4 bond, and wherein,
the dermatane sulfate is decomposed by heating in hydrogen chloride methanol solution containing 2,2-dimethoxypropane, at a temperature of 60 to 80°C, and for 20 to 100 minutes, and
the heparan sulfate is decomposed by the method according to claim 1 or 2.

**10.** The method according to claim 9, wherein the heating for the decomposition of the dermatan sulfate is performed at a temperature of 63 to 67°C and for 30 to 80 minutes.

**11.** The method according to claim 9 or 10, wherein the sample is selected from or derived from any one of a bodily fluid, a cell, a tissue, an organ, a cell culture, a tissue culture, a food, and a feedstuff.

**12.** The method according to claim 9 or 10, wherein the sample is selected from or derived from the group consisting of a bodily fluids, a cell, a tissue, an organ, a blood, a serum, a plasma, a urine, a bone marrow fluid, and a cerebrospinal fluid, obtained from a mammal.

**13.** The method according to claim 12, wherein the mammal is selected from the group consisting of a human, a monkey, a mouse, a rat, a guinea pig, a hamster, a rabbit, a horse, a bovine, a pig, a dog, and a cat.

**14.** The method according to claim 12, wherein the mammal is a human, and the human is a patient with a disease in which the heparan sulfate or/and the dermatan sulfate accumulate in a body.

**15.** The method according to claim 14, wherein the disease is selected from the group consisting of Hunter syndrome, Hurler syndrome, Scheie syndrome, Hurler-Scheie syndrome, Maroteaux-Lamy syndrome, Sanfilippo syndrome, and Sly syndrome.

**16.** The method according to claim 14 or 15, wherein the patient has been treated to reduce the heparan sulfate or/and the dermatan sulfate accumulated in the body.

**17.** A method for measuring the amount of the heparan sulfate in the sample, the method comprising:

the step of subjecting the disaccharide obtained by the methods according to any one of claims 1 to 8 to liquid chromatography to obtain an eluate, and
the step of subjecting the eluate to mass spectrometry.

**18.** A method for determining the amounts of the heparan sulfate and the dermatan sulfate in the sample, the method comprising:

the step of subjecting the disaccharide obtained, by the methods according to any one of claims 9 to 16, by decomposing the dermatan sulfate and the heparan sulfate, respectively, to obtain an eluate, and
the step of subjecting the eluate to mass spectrometry.

**19.** A method of detecting an individual with a disease in which heparan sulfate or/and dermatan sulfate accumulate in a body from among mammals from which the sample is provided, based on a measured value, obtained by the method according to claim 17 or 18, of the heparan sulfate or/and the dermatan sulfate contained in the sample.

**20.** The method of claim 19, wherein the disease is selected from the group consisting of Hunter syndrome, Hurler syndrome, Scheie syndrome, Hurler-Scheie syndrome, Maroteaux-Lamy syndrome, Sanfilippo syndrome, and Sly syndrome.

**21.** A method of confirming the effect of a treatment for decreasing heparan sulfate and dermatan sulfate accumulated in a body, based on a measured value, obtained by the method according to claim 17 or 18, of the heparan sulfate or/and the dermatan sulfate contained in a sample, the sample is obtained from a patient with a disease in which heparan sulfate and dermatan sulfate accumulate in the body before and after the treatment, respectively.

[Figure 1]

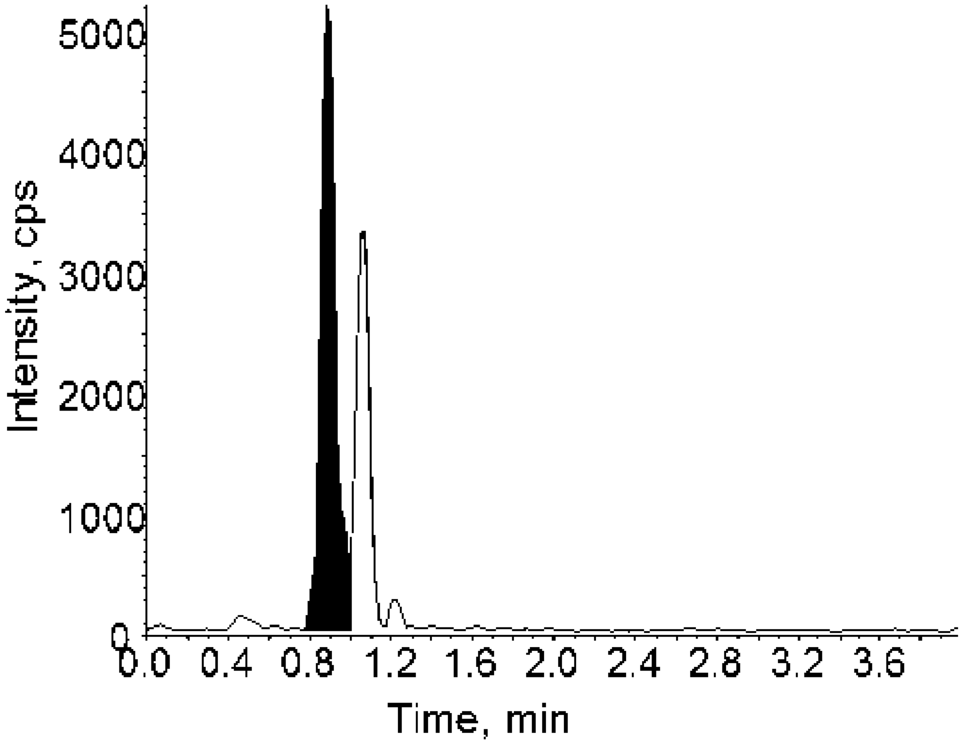


[Figure 2]

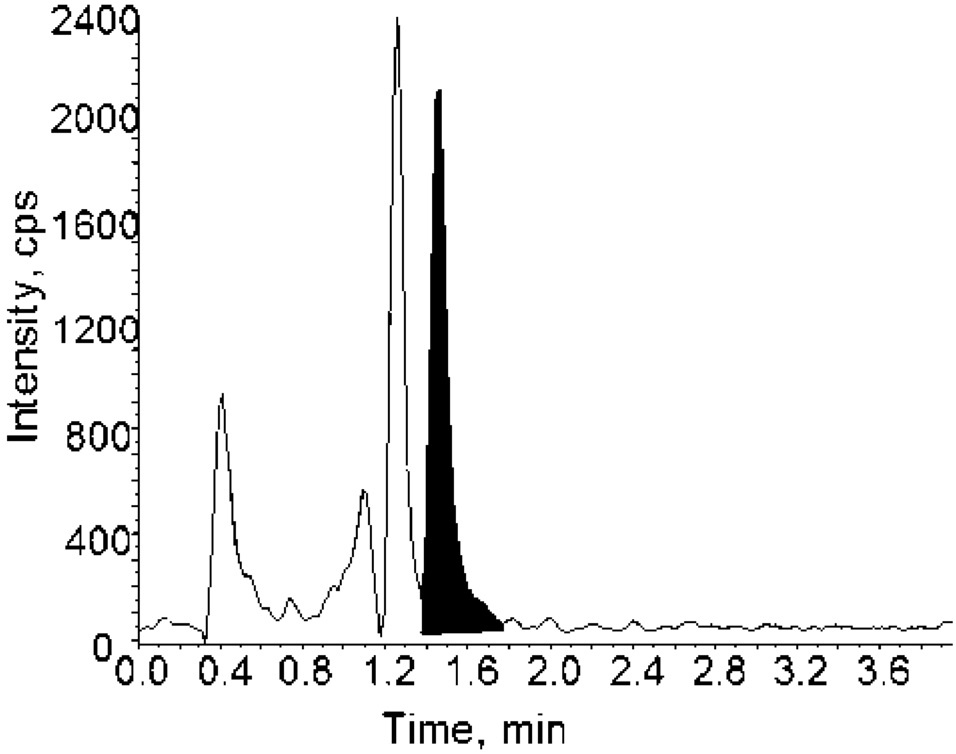


[Figure 3]

41

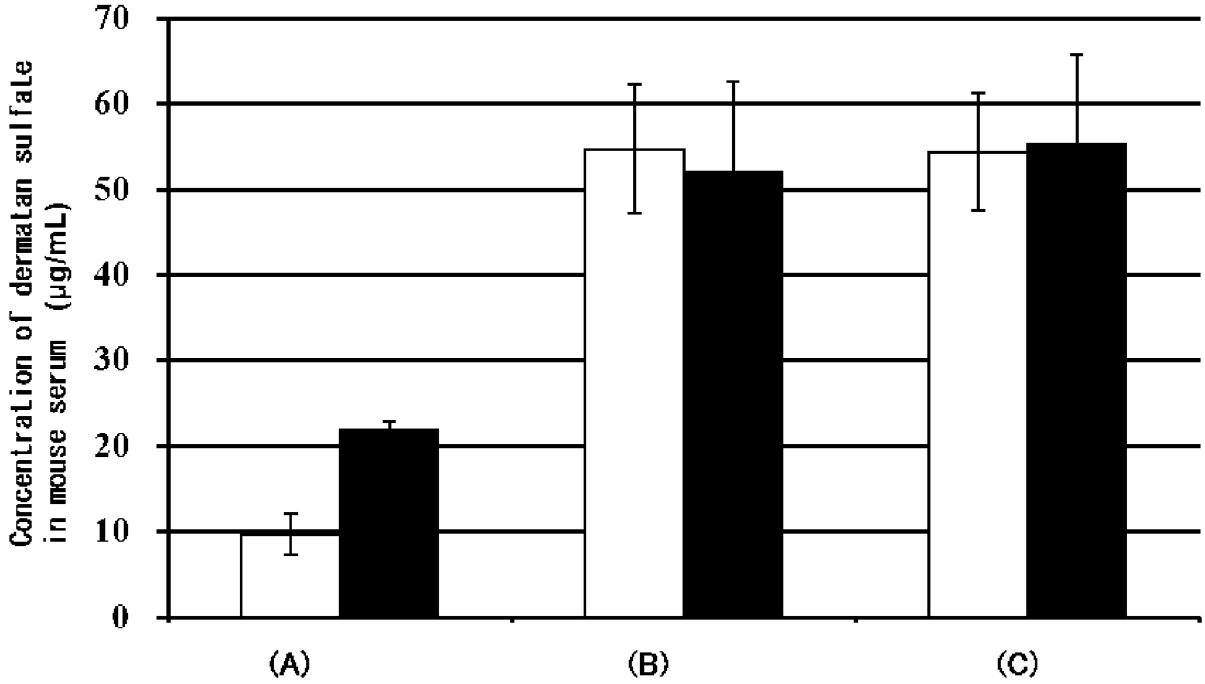


[Figure 4]

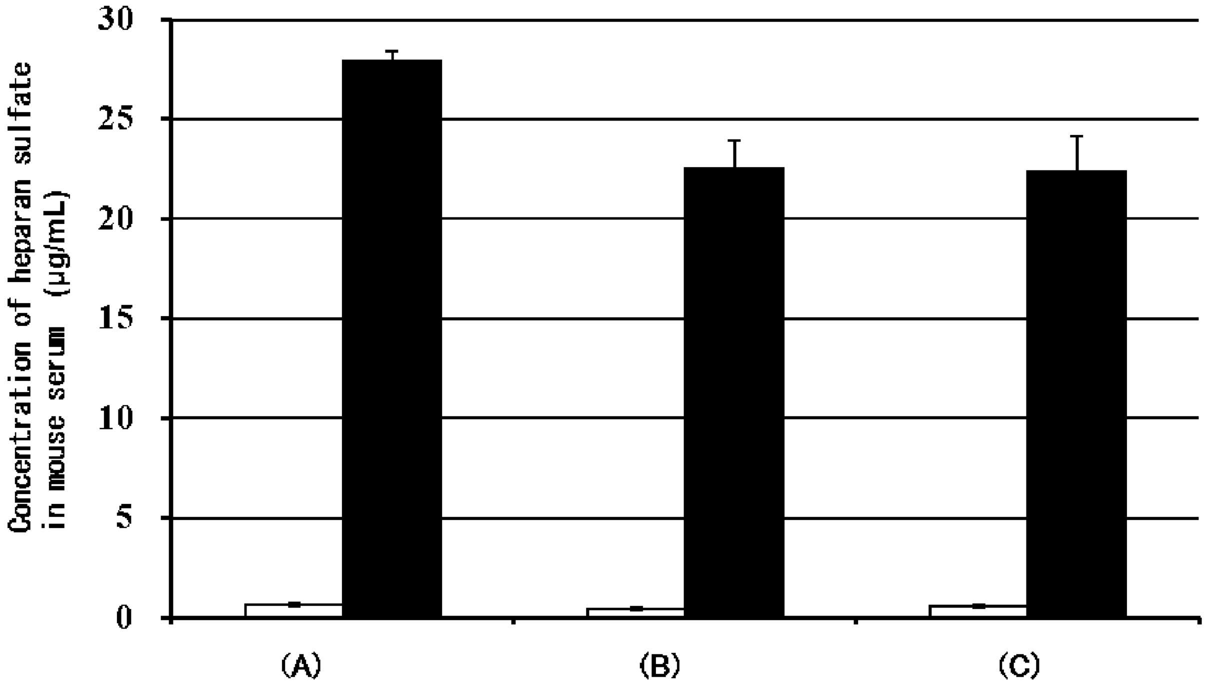


[Figure 5]

42

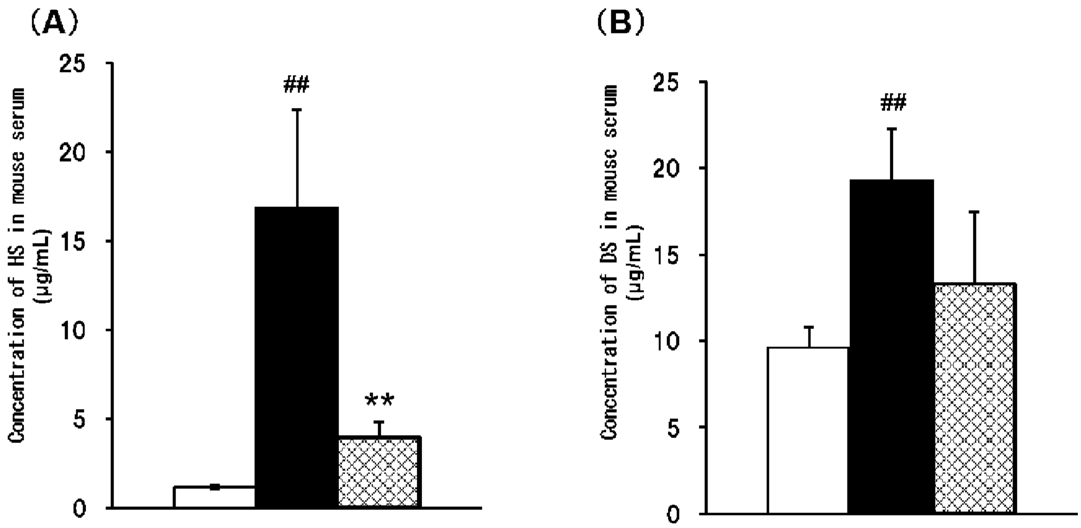
(A)
Concentration of HS in mouse serum (μg/mL)
25
20
15
10
5
0
##
**
(B)
Concentration of DS in mouse serum (μg/mL)
25
20
15
10
5
0
##

**INTERNATIONAL SEARCH REPORT**

International application No.
PCT/JP2018/006043

A. CLASSIFICATION OF SUBJECT MATTER
Int.Cl. G01N33/66(2006.01)i, G01N27/62(2006.01)i, G01N30/06(2006.01)i, G01N30/88(2006.01)i, G01N33/48(2006.01)i

According to International Patent Classification (IPC) or to both national classification and IPC

B. FIELDS SEARCHED
Minimum documentation searched (classification system followed by classification symbols)
Int.Cl. G01N33/66, G01N27/62, G01N30/06, G01N30/88, G01N33/48

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| A | AURAY-BLAIS, C. et al., Efficient analysis of urinary glycosaminoglycans by LC-MS/MS in mucopolysaccharidoses type I, II and VI, Molecular Genetics and Metabolism, January 2011, vol. 102, issue 1, 49-56 | 1-21 |
| A | AURAY-BLAIS, C. et al., An improved method for glycosaminoglycan analysis by LC-MS/MS of urine samples collected on filter paper, Clinica Chimica Acta, 11 April 2012, vol. 413, issues 7, 8, 771-778 | 1-21 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05.04.2018 | 24.04.2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/006043

C (Continuation). DOCUMENTS CONSIDERED TO BE RELEVANT

| Category* | Citation of document, with indication, where appropriate, of the relevant passages | Relevant to claim No. |
|---|---|---|
| A | ZHANG, H. Y. et al., Analysis of glycosaminoglycans in cerebrospinal fluid from patients with mucopolysaccharidoses by isotope-dilution ultra-performance liquid chromatography-tandem mass spectrometry, Clinical Chemistry, July 2011, vol. 57, issue 7, 1005-1012 | 1-21 |
| A | TOIDA, T. et al., Gas chromatography-mass spectrometric determinations of iduronic and glucuronic acids in glycosaminoglycans after reduction of carboxylic group using sodium borodeuteride, Analytical Sciences, December 1992, vol. 8, 799-804 | 1-21 |
| A | JP 2004-168771 A (SEIKAGAKU CORPORATION) 17 June 2004, paragraphs [0012], [0013] (Family: none) | 1-21 |
| A | JP 2004-210714 A (SEIKAGAKU CORPORATION) 29 July 2004, paragraph [0030] (Family: none) | 1-21 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012108056 A


### Non-patent literature cited in the description


- **AURAY-BLAIS C.** *Mol Genet Metab.,* 2011, vol. 102, 49-56
- **AURAY-BLAIS C.** *Clin Chim Acta.,* 2012, vol. 413, 771-8
- **ZANG H.** *Clin Chem.,* 2011, vol. 57, 1005-12